# EUROPÄISCHE PATENTSCHRIFT

(11) **EP 2 566 616 B1**
(45) Veröffentlichungstag und Bekanntmachung des Hinweises auf die Patenterteilung: **10.06.2015**
(21) Anmeldenummer: 11719767.3
(22) Anmeldetag: 29.04.2011
(51) Int. Cl.: B01F 17/14, B01F 17/34, A61K 8/55

(54) **ZUSAMMENSETZUNGEN ENTHALTEND PHOSPHORSÄUREALKYLESTER UND POLYOLESTER**
COMPOSITIONS CONTAINING PHOSPHORIC ACID ALKYL ESTERS AND POLYOL ESTERS
COMPOSITIONS CONTENANT DES ALKYLESTERS D'ACIDE PHOSPHORIQUE ET DES POLYESTERS

(30) Priorität: 06.05.2010 DE 102010019506
(43) Veröffentlichungstag der Anmeldung: 13.03.2013
(73) Patentinhaber: Clariant Finance (BVI) Limited, Road Town, Tortola (VG)
(72) Erfinder: STELTER, Wibke, 61191 Rodheim vor der Höhe (DE); SIEFER, Beate, 65931 Frankfurt/Main (DE); GEHM, Sonja, 65812 Bad Soden am Taunus (DE)
(74) Vertreter: Paczkowski, Marcus
(86) Internationale Anmeldenummer: PCT/EP2011/002160
(87) Internationale Veröffentlichungsnummer: WO 2011/137992

(56) Entgegenhaltungen:
- EP-A1- 0 924 217
- DE-A1- 10 308 565
- DE-A1- 10 353 030
- DE-A1- 19 707 800
- DE-A1-102005 051 222

## Beschreibung

Die Erfindung betrifft flüssige Zusammensetzungen enthaltend Phosphorsäurealkylester und Polyolester und die Verwendung dieser Zusammensetzungen zur Herstellung von kosmetischen, dermatologischen oder pharmazeutischen Formulierungen oder als Emulgator.

Die Verwendung von Emulgatoren zur Herstellung von Cremes, Lotionen, Salben etc., die mehrere nicht miteinander mischbare Substanzen (z. B. Wasser, Öl, organische und anorganische Bestandteile) enthalten, ist lange bekannt.

Als Emulgatoren werden beispielsweise auch Phosphorsäurealkylester verwendet wie beispielsweise in DE 197 07 800, EP 0 924 217, DE 103 08 565 und EP 0 901 811 offenbart. Bei Verwendung in kosmetischen, dermatologischen oder pharmazeutischen Formulierungen liegen Phosphorsäurealkylester in neutralisierter oder teilneutralisierter Form vor, da diese Formulierungen einen derartigen pH-Wert aufweisen, dass die Haut und/oder das Haar nicht geschädigt werden und dieser pH-Wert zur Neutralisation oder zumindest zur Teilneutralisation der Phosphorsäurealkylester führt. Man ist aus diesem Grund bei der Verwendung von Phosphorsäurealkylestern, z. B. bei ihrer Verwendung zur Herstellung von kosmetischen, dermatologischen oder pharmazeutischen Formulierungen, insbesondere an deren neutralisierten oder teilneutralisierten Formen interessiert und darüber hinaus an flüssigen Darreichungsformen, weil diese einfacher zu formulieren sind als Feststoffe.

Neutralisierte oder teilneutralisierte Phosphorsäurealkylester liegen jedoch zumeist in fester oder pastöser Form vor. Neutralisierte oder teilneutralisierte Phosphorsäureester, die flüssig sind, haben dagegen den Nachteil, dass sie oftmals ethoxyliert sind. Da ethoxylierte Substanzen im Verdacht stehen, die Haut durchlässiger für irritierende und allergene Schadstoffe zu machen und diese dadurch in den Körper einzuschleusen, sind diese in kosmetischen, dermatologischen oder pharmazeutischen Formulierungen oftmals unerwünscht.

Aufgabe der vorliegenden Erfindung war es daher, neutralisierte oder teilneutralisierte Phosphorsäurealkylester in flüssiger Form zur Verfügung zu stellen, die vorzugsweise auch frei sind von ethoxylierten Verbindungen.

Es wurde gefunden, dass diese Aufgabe durch spezielle Zusammensetzungen enthaltend bestimmte Phosphorsäurealkylester in neutralisierter oder teilneutralisierter Form und bestimmte Polyolester gelöst wird.

Gegenstand der Erfindung sind daher flüssige Zusammensetzungen enthaltend
a) einen oder mehrere Phosphorsäurealkylester der Formel (I) 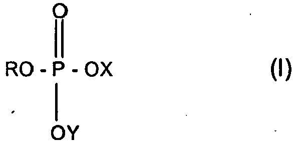 worin
   - R: verzweigtes C₁₂-C₂₄-Alkyl, vorzugsweise verzweigtes C₁₄-C₂₂-Alkyl, bedeutet,
   - X: H, Alkali oder Erdalkali bedeutet,
   - Y: verzweigtes C₁₂-C₂₄-Alkyl, vorzugsweise verzweigtes C₁₄-C₂₂-Alkyl, oder H, Alkali oder Erdalkali bedeutet,
   und der Neutralisationsgrad der nicht veresterten Gruppen OX und OY gemeinsam (bezogen auf die Summe aller nicht veresterten Gruppen OX und OY) mindestens 25 mol-% ist, und
b) einen oder mehrere Polyolester, die erhältlich sind aus der Reaktion eines Polyols mit einer oder mehreren Säuren, wobei das Polyol ausgewählt ist aus gesättigten Polyolen bestehend aus Kohlenstoff-, Wasserstoff- und Sauerstoffatomen mit 3 bis 6 Kohlenstoffatomen und 2 bis 6 OH-Gruppen sowie Oligomeren davon mit im Mittel 1 bis 4 Wiederholungseinheiten und die eine oder die mehreren Säuren ausgewählt sind aus Carbonsäuren R¹-COOH, wobei R¹ ein linearer oder verzweigter gesättigter Alkylrest mit 7 bis 29 Kohlenstoffatomen oder ein linearer oder verzweigter ein- oder mehrfach ungesättigter Alkenylrest mit 7 bis 29 Kohlenstoffatomen ist und maximal 50 mol-% der OH-Gruppen des Polyols verestert sind, und
wobei die Komponenten a) und b) zusammen zu mindestens 80 Gew.-% in den flüssigen Zusammensetzungen enthalten sind, bezogen auf das Gesamtgewicht der flüssigen Zusammensetzungen, und das Gewichtsverhältnis von Verbindungen der Komponente a), bezogen auf die nicht neutralisierte Form, zu Verbindungen der Komponente b) von 12,5 : 87,5 bis 87,5 : 12,5 ist.

"Flüssige (erfindungsgemäße) Zusammensetzungen" bedeutet im Rahmen der vorliegenden Erfindung, dass die Zusammensetzungen bei Raumtemperatur (25 °C) und einem Druck von 1013 mbar (1,013 · 105 Pa) flüssig sind.

Wie bereits erwähnt, ist in den Phosphorsäurealkylestern der Formel (I) der Neutralisationsgrad der nicht veresterten Gruppen OX und OY gemeinsam (bezogen auf die Summe aller nicht veresterten Gruppen OX und OY) mindestens 25 mol%. Die Gruppen OX sind immer nicht verestert. Die Gruppen OY sind immer dann nicht verestert, wenn Y nicht verzweigtes C₁₂-C₂₄-Alkyl bedeutet. In den nicht veresterten Gruppen OX und OY können X und Y demnach H, Alkali oder Erdalkali bedeuten. Der Neutralisationsgrad der nicht veresterten Gruppen OX und OY gemeinsam (bezogen auf die Summe aller nicht veresterten Gruppen OX und OY) von mindestens 25 mol-% bedeutet, dass X und Y zusammen in mindestens 25 mol-% der Summe aller nicht veresterten Gruppen OX und OY eine andere Bedeutung als H haben, also Alkali oder Erdalkali bedeuten.

Die erfindungsgemäßen Zusammensetzungen zeichnen sich durch eine starke Erniedrigung der Grenzflächenspannung auch bei hoher Temperaturbelastung gegenüber polaren und unpolaren Bestandteilen aus. Die erfindungsgemäßen Zusammensetzungen zeigen eine verbesserte Stabilität gegenüber Elektrolytzusätzen und Säuren und eine hohe Lagerstabilität. Sie liegen vorzugsweise im pH-Bereich von 5 bis 7 vor und können somit als sehr hautfreundliche Emulgatoren sowohl in Öl-in-Wasser als auch in Wasser-in-Öl-Emulsionen eingesetzt werden, bevorzugt in Hautpflegemitteln. Durch die flüssige Konsistenz der erfindungsgemäßen Zusammensetzungen ist zum einen ein energieintensives Aufschmelzen der Emulgatorkomponente nicht notwendig, wodurch die Herstellung von Emulsionen bei niedrigeren Temperaturen wie z. B. Raumtemperatur ermöglicht ist.

In DE 197 07 800 werden Mischungen aus Mono-, Di- und Trialkylmonophosphorsäureestern beschrieben, wobei die Alkylgruppen beta-verzweigte Alkylgruppen und die nicht veresterten Gruppen mit Alkali oder Erdalkali neutralisiert sein können. Die Mischungen können als Emulgatoren verwendet werden, beispielsweise in kosmetischen oder pharmazeutischen Erzeugnissen. In der DE 197 07 800 werden dagegen keine bei Raumtemperatur flüssigen Zusammensetzungen beschrieben, die zumindest teilneutralisierte Phosphorsäurealkylester mit verzweigten Alkylgruppen und einem Neutralisationsgrad von mindestens 25 mol-% sowie Polyolester enthalten und wobei die Phosphorsäurealkylester in einer Menge von mindestens 10 Gew.-% und die Summe aus Phosphorsäurealkylester und Polyolester zusammen in einer Menge von mindestens 80 Gew.-% in den Zusammensetzungen enthalten sind.

In EP 0 924 217 werden Phosphorsäurealkylester offenbart, wobei die Alkylgruppen beispielsweise Mischungen aus geradkettigen und beta-verzweigten Alkylgruppen sein können und die nicht veresterten Gruppen mit Stickstoffhaltigen Gegenionen neutralisiert sind. Die Phosphorsäurealkylester können beispielsweise als Emulgatoren für wasserhaltige Emulsionen verwendet werden.

In EP 0 901 811 werden Mischungen von langkettigen Phosphorsäurealkylestern beschrieben, wobei die Alkylreste Mischungen aus geradkettigen und beta-verzweigten Alkylresten sind und die nicht veresterten Gruppen mit Alkali oder Erdalkali neutralisiert sein können. Diese langkettigen Phosphorsäurealkylester eignen sich als Emulgatoren insbesondere für Öl-in-Wasser Emulsionen kosmetischer oder pharmazeutischer Art.

Die Herstellung der Säureform der Phosphorsäurealkylester der Komponente a) erfolgt nach an sich bekannten Verfahren, beispielsweise durch Umsetzung von Tetraphosphordecaoxid mit verzweigten Alkoholen mit C₁₂-C₂₄-Alkylgruppen. Bei diesem Herstellverfahren fallen die Phosphorsäureester als ein Gemisch aus im Wesentlichen Mono- und Diester mit geringen Anteilen an Triester an. Die neutralisierte oder teilneutralisierte Form der Phosphorsäurealkylester kann nach dem Fachmann geläufigen Methoden aus der Säureform durch Umsetzung mit geeigneten Basen erhalten werden. In einer bevorzugten Ausführungsform findet diese Neutralisation in Gegenwart des Polyolesters der Komponente b) statt, wobei erfindungsgemäße Zusammensetzungen erhalten werden können.

Bei den verzweigten Alkoholen aus denen der Rest R und gegebenenfalls auch der Rest Y hervorgeht, handelt es sich vorzugsweise um beta-verzweigte Alkohole bzw. um sogenannte Guerbetalkohole, die durch die Guerbet-Synthese zugänglich sind (Ullmann's Enzyclopedia of Industrial Chemistry, 5th Edition, Vol. A 10, p. 88).

Die Herstellung der Polyolester der Komponente b) kann in einfacher Weise nach den dem Fachmann geläufigen Methoden erfolgen. Die Polyolester sind darüber hinaus käuflich erwerbbar.

In einer bevorzugten Ausführungsform der Erfindung bedeutet in dem einen oder den mehreren Phosphorsäurealkylestern der Komponente a) der erfindungsgemäßen Zusammensetzungen R verzweigtes C₂₀-Alkyl, X H oder Kalium und Y verzweigtes C₂₀-Alkyl, H oder Kalium.

In einer weiteren bevorzugten Ausführungsform der Erfindung ist in dem einen oder den mehreren Phosphorsäurealkylestern der Komponente a) der erfindungsgemäßen Zusammensetzungen R beta-verzweigtes Alkyl und Y, sofern es Alkyl bedeutet, ebenfalls beta-verzweigtes Alkyl.

Vorzugsweise ist das Gewichtsverhältnis von Phosphorsäuremonoalkylester zu Phosphorsäuredialkylester in der Komponente a) von 1 : 2 bis 2 : 1. Besonders bevorzugt ist dieses Gewichtsverhältnis von 1 : 1,5 bis 1,5 :1.

Vorzugsweise ist der Neutralisationsgrad der nicht veresterten Gruppen OX und OY gemeinsam (bezogen auf die Summe aller nicht veresterten Gruppen OX und OY) in dem einen oder den mehreren Phosphorsäurealkylestern der Formel (I) mindestens 30 mol-% und besonders bevorzugt mindestens 50 mol-%. Vorzugsweise ist der Neutralisationsgrad der nicht veresterten Gruppen OX und OY gemeinsam (bezogen auf die Summe aller nicht veresterten Gruppen OX und OY) in dem einen oder den mehreren Phosphorsäurealkylestern der Formel (I) maximal 90 mol-%.

In einer weiteren bevorzugten Ausführungsform der vorliegenden Erfindung ist R¹ in dem einen oder den mehreren Polyolestern der Komponente b) der erfindungsgemäßen Zusammensetzungen ein linearer oder verzweigter gesättigter Alkylrest mit 7 bis 29 und vorzugsweise mit 7 bis 17 Kohlenstoffatomen oder ein linearer oder verzweigter ein- oder mehrfach ungesättigter Alkenylrest mit 7 bis 29 und vorzugsweise mit 7 bis 17 Kohlenstoffatomen.

In einer besonders bevorzugten Ausführungsform der vorliegenden Erfindung ist R¹ in dem einen oder den mehreren Polyolestern der Komponente b) der erfindungsgemäßen Zusammensetzungen ein linearer oder verzweigter gesättigter Alkylrest mit 7 bis 29 und vorzugsweise mit 7 bis 17 Kohlenstoffatomen.

In einer weiteren bevorzugten Ausführungsform der Erfindung ist der eine oder sind die mehreren Polyolester der Komponente b) der erfindungsgemäßen Zusammensetzungen ausgewählt aus der Gruppe bestehend aus Sorbitanestern, Glycerinestern und Oligo- oder Polyglycerinestern und vorzugsweise ausgewählt aus der Gruppe bestehend aus Sorbitanestern und Oligo- oder Polyglycerinestern.

In einer weiteren bevorzugten Ausführungsform der Erfindung ist der eine oder sind die mehreren Polyolester der Komponente b) der erfindungsgemäßen Zusammensetzungen ausgewählt aus der Gruppe bestehend aus Sorbitan Laurate (z. B. Tego^{®} SML), Sorbitan Isostearate (z. B. Span^{®} 120), Sorbitan Diisostearate, Sorbitan Sesquiisostearate, Sorbitan Palmitate (z. B. Span^{®} 40), Sorbitan Stearate (z. B. Span^{®} 60), Sorbitan Distearate, Sorbitan Sesquistearate, Sorbitan Caprylate, Sorbitan Sesquicaprylate (z. B. Antil ^{®}Soft SC), Sorbitan Cocoate, Sorbitan Oleate (z. B. Tego SMO V), Sorbitan Dioleate, Sorbitan Sesquioleate, Sorbitan Palmate, Sorbitan Olivate (z. B. Olivem^{®} 900), Glyceryl Laurate (z. B. MONOMULS^{®} 90-L-12), Glyceryl Stearate (z. B. Cithrol^{®} GMS), Polyglyceryl-2 Sesquiisostearate (z. B. Hostacerin^{®} DGI), Polyglyceryl-2 Caprate (z. B. Demosoft^{®} DGMC), Polyglyceryl-2 Caprylate, Polyglyceryl-2 Diisostearate, Polyglyceryl-2 Dioleate, Polyglyceryl-2 Distearate, Polyglyceryl-2 Isostearate (z. B. Cithrol™ PG21IS), Polyglyceryl-2 Laurate (z. B. Dermofeel^{®} G 2 L), Polyglyceryl-2 Myristate, Polyglyceryl-2 Oleate, Polyglyceryl-2 Palmitate, Polyglyceryl-3 Caprate (z. B. Tegosoft^{®} PC 31), Polyglyceryl-3 Caprylate (z. B. Tego^{®} Cosmo P 813), Polyglyceryl-3 Cocoate, Polyglyceryl-3 Dicocoate, Polyglyceryl-3 Diisostearate (z. B. Lameform^{®} TGI), Polyglyceryl-3 Dioleate (z. B. Plurol^{®} Pleique CC 497), Polyglyceryl-3 Distearate (z. B. Cremophor^{®} GS 32), Polyglyceryl-3 Isostearate, Polyglyceryl-3 Laurate (z. B. Hydramol^{®} TGL Ester), Polyglyceryl-3 Myristate, Polyglyceryl-3 Oleate (z. B. Cremophor^{®} TGO), Polyglyceryl-3 Palmitate (z. B. Dermofeel^{®} PP), Polyglyceryl-4 Caprate (z. B. Tegosoft^{®} PC 41), Polyglyceryl-4 Caprylate, Polyglyceryl-4 Dilaurate, Polyglyceryl-4 Laurate (z. B. Tego^{®} Care PL 4) und Polyglyceryl-4 Oleate.

In dem vorhergehenden Absatz sind die Polyolester der Komponente b) der erfindungsgemäßen Zusammensetzungen mit ihren INCI-Namen bezeichnet.

In einer besonders bevorzugten Ausführungsform der Erfindung ist der Polyolester der Komponente b) der erfindungsgemäßen Zusammensetzungen Sorbitanmonolaurat oder Sorbitanmonocaprylat.

Enthalten die erfindungsgemäßen Zusammensetzungen Sorbitanmonocaprylat, kann dieses die benötigte Menge an Konservierungsmittel in den kosmetischen Formulierungen, in denen diese erfindungsgemäßen Zusammensetzungen eingesetzt werden, herabsetzen.

Vorzugsweise ist in den erfindungsgemäßen Zusammensetzungen das Gewichtsverhältnis von Verbindungen der Komponente a), bezogen auf die nicht neutralisierte Form, zu Verbindungen der Komponente b) von 30 : 70 bis 70 : 30. Besonders bevorzugt ist dieses Gewichtsverhältnis von 40 : 60 bis 60 : 40.

In einer weiteren bevorzugten Ausführungsform der Erfindung enthalten die erfindungsgemäßen Zusammensetzungen, bezogen auf das Gesamtgewicht der Zusammensetzungen, 0 bis 5 Gew.-% und vorzugsweise 0,05 bis 5 Gew.-% eines oder mehrerer Triester der Formel (II) 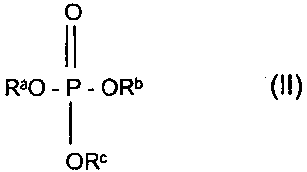 worin R^{a}, R^{b} und R^{c} jeweils unabhängig voneinander verzweigtes C₁₂-C₂₄-Alkyl, vorzugsweise verzweigtes C₁₄-C₂₂-Alkyl, bedeuten.

In einer weiteren bevorzugten Ausführungsform der Erfindung enthalten die erfindungsgemäßen Zusammensetzungen Wasser, vorzugsweise in einer Menge von mindestens 2,0 Gew.-%, bezogen auf das Gesamtgewicht der Zusammensetzungen.

Sofern Wasser in den erfindungsgemäßen Zusammensetzungen enthalten ist, ist die Menge an Wasser, bezogen auf das Gesamtgewicht der Zusammensetzungen, vorzugsweise kleiner oder gleich 10,0 Gew.-% und besonders bevorzugt ist die Menge an Wasser von 2,0 bis 8,0 Gew.-%, bezogen auf das Gesamtgewicht der Zusammensetzungen.

Die erfindungsgemäßen Zusammensetzungen können weitere Hilfs- und Zusatzstoffe enthalten wie z. B. Öle, Tenside, Wachse, Emulgatoren, Co-Emulgatoren, Solubilisatoren, Elektrolyte, Hydroxysäuren, Stabilisatoren, antimikrobielle Wirkstoffe, Antioxidantien und/oder Lösungsmittel. Diese weiteren Hilfs- und Zusatzstoffe können beispielsweise solche Substanzen sein, die üblicherweise auch in kosmetischen, dermatologischen oder pharmazeutischen Formulierungen verwendet werden. Beispiele für solche Hilfs- und Zusatzstoffe sind im Folgenden im Rahmen der Beschreibung von kosmetischen, dermatologischen oder pharmazeutischen Formulierungen aufgeführt. Die gegebenenfalls in den erfindungsgemäßen Zusammensetzungen enthaltenen weiteren Hilfs- und Zusatzstoffe sind jedoch von den Phosphorsäurealkylestern der Komponente a) und von den Polyolestern der Komponente b) verschieden. Die Menge der gegebenenfalls in den erfindungsgemäßen Zusammensetzungen enthaltenen weiteren Hilfs- und Zusatzstoffe ist vorzugsweise von 0 bis 10 Gew.-% und besonders bevorzugt von 0,1 bis 10 Gew.-%, bezogen auf das Gesamtgewicht der Zusammensetzungen.

In einer weiteren bevorzugten Ausführungsform der Erfindung können die erfindungsgemäßen Zusammensetzungen ein oder mehrere Alkohole enthalten. Vorzugsweise sind diese Alkohole ausgewählt aus gesättigten Alkoholen mit einer verzweigten C₁₂-C₂₄-Alkylgruppe, besonders bevorzugt mit einer verzweigten C₁₄-C₂₂-Alkylgruppe. Die Menge dieser gegebenenfalls in den erfindungsgemäßen Zusammensetzungen enthaltenen Alkohole ist vorzugsweise von 0 bis 10 Gew.-% und besonders bevorzugt von 0,1 bis 8 Gew.-%, bezogen auf das Gesamtgewicht der Zusammensetzungen.

Weiterhin bevorzugt sind die erfindungsgemäßen Zusammensetzungen frei von ethoxylierten Verbindungen.

In einer besonders bevorzugten Ausführungsform der Erfindung enthalten die flüssigen Zusammensetzungen
a) einen oder mehrere Phosphorsäurealkylester der Formel (I) 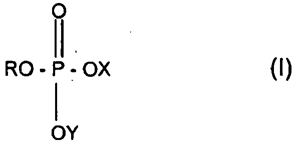 worin
   - R: verzweigtes C₁₈₋₂₂-Alkyl und vorzugsweise C₂₀-Alkyl bedeutet,
   - X: H oder Kalium bedeutet,
   - Y: verzweigtes C₁₈₋₂₂-Alkyl und vorzugsweise C₂₀-Alkyl, oder H oder Kalium bedeutet,
   und der Neutralisationsgrad der nicht veresterten Gruppen OX und OY gemeinsam (bezogen auf die Summe aller nicht veresterten Gruppen OX und OY) 50 bis 70 mol-% und vorzugsweise 60 mol-% ist, und
b) Sorbitanmonolaurat oder Sorbitanmonocaprylat
wobei die Komponenten a) und b) zusammen zu mindestens 90 Gew.-% in den flüssigen Zusammensetzungen enthalten sind, bezogen auf das Gesamtgewicht der flüssigen Zusammensetzungen, und das Gewichtsverhältnis von Verbindungen der Komponente a), bezogen auf die nicht neutralisierte Form, zu Verbindungen der Komponente b) von 30 : 70 bis 70 : 30 und vorzugsweise 50 : 50 ist.

Die erfindungsgemäßen Zusammensetzungen weisen vorzugsweise Viskositäten von 500 bis 25000 mPa · s und besonders bevorzugt von 1000 bis 20000 mPa · s auf. Die Viskositäten werden mit einem Brookfield Viskosimeter Typ RDV-I+ gemessen (20 °C; 20 Umdrehungen pro Minute; je nach Viskosität RV-Spindel Nr. 4 - 6).

Die erfindungsgemäßen Zusammensetzungen sind in vorteilhafter Weise zur Herstellung von kosmetischen, dermatologischen oder pharmazeutischen Formulierungen geeignet.

Ein weiterer Gegenstand der vorliegenden Erfindung betrifft daher die Verwendung einer erfindungsgemäßen Zusammensetzung zur Herstellung von kosmetischen, dermatologischen oder pharmazeutischen Formulierungen.

Diese kosmetischen, dermatologischen oder pharmazeutischen Formulierungen können als weitere Hilfs- und Zusatzstoffe Öle, Tenside, Wachse, Emulgatoren, Co-Emulgatoren, Solubilisatoren, Elektrolyte, Hydroxysäuren, Stabilisatoren, kationische Polymere, Filmbildner, Verdicker, Gelierungsmittel, Überfettungsmittel, Rückfetter, antimikrobielle Wirkstoffe, biogene Wirkstoffe, Adstringentien, deodorierende Stoffe, Sonnenschutzfilter, Antioxidantien, Feuchthaltemittel, organische Lösungsmittel, Farbmittel, Perlglanzmittel, Duftstoffe, Trübungsmittel, wasserlösliche Silikone und/oder Wasser enthalten.

Die Öle können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Methanol, Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318 sowie das Handelsprodukt Velsan^{®} CCT (Capryl-Caprinsäure-Triglycerid, Clariant). Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈₋₂₂-Alkanolen, z. B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether, Di-3-ethyldecylether, tert.-Butyl-n-octylether, iso-Pentyl-n-octylether und 2-Methyl-pentyl-n-octylether sowie Di-tert.-butylether und Di-iso-pentylether.

Ebenso in Betracht kommen verzweigte gesättigte oder ungesättigte Fettalkohole mit 6 - 30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich-primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z. B. unter dem Handelsnamen Cosmacol^{®} der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglykol-dioleat, Ethylenglykol-di-isotridecanoat, Propylenglykol-di-(2-ethylhexanoat), Propylenglykol-di-isostearat, Propylenglykol-di-pelargonat, Butandiol-di-isostearat und Neopentylglykoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®}CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Chofesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®}S), Ozokerit, und Ceresin.

Ebenso in Betracht kommen Silikonöle bzw. -wachse, vorzugsweise Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41 M65, SilCare^{®} Silicone 41M70, SilCare^{®} Silicone 41M80 (Clariant) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SiICare^{®} Silicone 41M40, SilCare^{®} Silicone 41M50 (Clariant) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die Menge an Öl in den kosmetischen, dermatologischen oder pharmazeutischen Formulierungen beträgt bevorzugt von 5 bis 80 Gew.-%, besonders bevorzugt von 10 bis 50 Gew.-% und insbesondere bevorzugt von 15 bis 30 Gew.-%, bezogen auf das Gesamtgewicht der Formulierungen.

Die kosmetischen, dermatologischen oder pharmazeutischen Formulierungen können kationische, nichtionische, ampholytische Tenside, Betaintenside und/oder in geringen Mengen anionische Tenside enthalten.

Die Gesamtmenge der in den kosmetischen, dermatologischen oder pharmazeutischen Formulierungen (z. B. im Falle von Rinse-Off-Produkten) eingesetzten Tenside beträgt, bezogen auf das Gesamtgewicht der Formulierungen, bevorzugt von 1,0 bis 70,0 Gew.-%, besonders bevorzugt von 5,0 bis 40,0 Gew.-% und insbesondere bevorzugt von 10,0 bis 35,0 Gew.-%.

Als anionische Tenside bevorzugt sind (C₁₀-C₂₂)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den kosmetischen, dermatologischen oder pharmazeutischen Formulierungen beträgt bevorzugt von 0,1 bis 10,0 Gew.-%, besonders bevorzugt von 0,2 bis 5,0 Gew.-% und insbesondere bevorzugt von 0,5 bis 2,0 Gew.-%, bezogen auf das Gesamtgewicht der Formulierungen.

Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyl-dimethylammoniumchlorid oder -bromid; (C₈-C₂₂)-Alkyl-dimethyl-ethylammoniumchlorid oder -bromid; (C₈-C₂₂)-Alkyl-trimethylammoniumchlorid oder -bromid, vorzugsweise Cetyltrimethylammoniumchlorid oder -bromid und (C₈-C₂₂)-Alkyl-trimethylammoniumchlorid oder -bromid; (C₁₀-C₂₄)-Alkyl-dimethylbenzyl-ammoniumchlorid oder -bromid, vorzugsweise (C₁₂-C₁₈)-Alkyl-dimethylbenzyl-ammoniumchlorid, (C₈-C₂₂)-Alkyl-dimethyl-hydroxyethylammoniumchlorid, -phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat.

Die Menge der kationischen Tenside in den kosmetischen, dermatologischen oder pharmazeutischen Formulierungen beträgt bevorzugt von 0,1 bis 10,0 Gew.-%, besonders bevorzugt von 0,5 bis 7,0 Gew.-% und insbesondere bevorzugt von 1,0 bis 5,0 Gew.-%, bezogen auf das Gesamtgewicht der Formulierungen.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den kosmetischen, dermatologischen oder pharmazeutischen Formulierungen (z. B. im Falle von Rinse-off-Produkten) liegt bevorzugt im Bereich von 1,0 bis 20,0 Gew.-%, besonders bevorzugt von 2,0 bis 10,0 Gew.-% und insbesondere bevorzugt von 3,0 bis 7,0 Gew.-%, bezogen auf das Gesamtgewicht der Formulierungen.

Weiterhin können die kosmetischen, dermatologischen oder pharmazeutischen Formulierungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze. Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈- Alkyldimethylaminoxide, Fettsäureamidoalkyldimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimetylbetaine.

Die Menge der amphoteren Tenside und/oder Betaintenside in den kosmetischen, dermatologischen oder pharmazeutischen Formulierungen beträgt bevorzugt von 0,5 bis 20,0 Gew.-% und besonders bevorzugt von 1,0 bis 10,0 Gew.-%, bezogen auf das Gesamtgewicht der Formulierungen.

Bevorzugte Tenside sind Laurylsulfat, Laurethsulfat, Cocoamidopropylbetain, Alkylbetaine wie Coco-Betain, Natriumcocoylglutamat und Lauroamphoacetat.

In einer weiteren bevorzugten Ausführungsform der Erfindung enthalten die kosmetischen, dermatologischen oder pharmazeutischen Formulierungen zusätzlich noch als schaumverstärkende Mittel Co-Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die kosmetischen, dermatologischen oder pharmazeutischen Formulierungen können Wachse, beispielsweise Paraffinwachse, Mikrowachse und Ozokerite, Bienenwachs und ihre Teilfraktionen sowie der Bienenwachsderivate, Wachse aus der Gruppe der homopolymeren Polyethylene oder Coplymere der α-Olefine, sowie natürliche Wachse wie Reiswachs, Candellilawachs, Carnaubawachs, Japanwachs oder Schellackwachs enthalten.

Die Menge an Wachs in den kosmetischen, dermatologischen oder pharmazeutischen Formulierungen beträgt bevorzugt von 0 bis 10 Gew.-% und besonders bevorzugt von 1 bis 6 Gew.-%, bezogen auf das Gesamtgewicht der Formulierungen.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht: Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono-und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglykol(13)stearylether, Polyethylenglykol(14)stearylether, Polyethylenglykol(15)stearylether, Polyethylenglykol(16)stearylether, Polyethylenglykol(17)stearylether, Polyethylenglykol(18)stearylether, Polyethylenglykol(19)stearylether, Polyethylenglykol(20)stearylether, Polyethylenglykol(12)isostearylether, Polyethylenglykol(13)isostearylether, Polyethylenglykol(14)isostearylether, Polyethylenglykol(15)isostearylether, Polyethylenglykol(16)isostearylether, Polyethylenglykol(17)isostearylether, Polyethylenglykol(18)isostearylether, Polyethylenglykol(19)isostearylether, Polyethylenglykol(20)isostearylether, Polyethylenglykol(13)cetylether, Polyethylenglykol(14)cetylether, Polyethylenglykol(15)cetylether, Polyethylenglykol(16)cetylether, Polyethylenglykol(17)cetylether, Polyethylenglykol(18)cetylether, Polyethylenglykol(19)cetylether, Polyethylenglykol(20)cetylether, Polyethylenglykol(13)isocetylether, Polyethylenglykol(14)isocetylether, Polyethylenglykol(15)isocetylether, Polyethylenglykol(16)isocetylether, Polyethylenglykol(17)isocetylether, Polyethylenglykol(18)isocetylether, Polyethylenglykol(19)isocetylether, Polyethylenglykol(20)isocetylether, Polyethylenglykol(12)oleylether, Polyethylenglykol(13)oleylether, Polyethylenglykol(14)oleylether, Polyethylenglykol(15)oleylether, Polyethylenglykol(12)laurylether, Polyethylenglykol(12)isolaurylether, Polyethylenglykol(13)cetylstearylether, Pölyethylenglykol(14)cetylstearylether, Polyethylenglykol(15)cetylstearylether, Polyethylenglykol(16)cetylstearylether, Polyethylenglykol(17)cetylstearylether, Polyethylenglykol(18)cetylstearylether, Polyethylenglykol(19)cetylstearylether.

Ebenso bevorzugt sind Fettsäureethoxylate, gewählt aus der Gruppe der ethoxylierten Stearate, Isostearate und Oleate, insbesondere Polyethylenglykol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders
Polyethylenglykol(20)sorbitanmonolaurat,
Polyethylenglykol(20)sorbitanmonostearat,
Polyethylenglykol(20)sorbitanmonoisostearat,
Polyethylenglykcol(20)sorbitanmonopalmitat,
Polyethylenglykol(20)sorbitanmonooleat.

Besonders vorteilhafte Coemulgatoren sind Glycerylmonostearat, Glycerylmonooleat, Diglycerylmonostearat, Glycerylisostearat, Polyglyceryl-3-oleat , Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat (PEG: Polyethylenklykol), Diisostearoylpolyglyceryl-3-diisostearat, Glykoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglykol(2)stearylether (Steareth-2), Alkylmethiconcopolyole und Alkyl-Dimethiconcopolyole, insbesondere Cetyldimethiconcopolyol, Laurylmethiconcopolyol.

Die kosmetischen, dermatologischen oder pharmazeutischen Formulierungen können einen oder mehrere der Emulgatoren, Co-Emulgatoren oder Solubilisatoren in Mengen von vorzugsweise 0,1 bis 20,0 Gew.-%, besonders bevorzugt 1,0 bis 15,0 Gew.-% und insbesondere bevorzugt 3,0 bis 10,0 Gew.-%, bezogen auf das Gesamtgewicht der Formulierungen, enthalten.

Als Elektrolyt zum Einsatz können kommen anorganische Salze, bevorzugt Ammonium- oder Metallsalze, besonders bevorzugt von Halogeniden, beispielsweise CaCl₂, MgCl₂, LiCl, KCl und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure oder Galacturonsäure.

Hierzu zählen auch Aluminiumsalze, bevorzugt Alurniniumchlorohydrat oder Aluminium-Zirkonium-Komplexsalze.

Als Elektrolyt können die kosmetischen, dermatologischen oder pharmazeutischen Formulierungen auch Mischungen verschiedener Salze enthalten. Der Gehalt an dem einen oder den mehreren Elektrolyten, bezogen auf das Gesamtgewicht der Formulierungen, ist vorzugsweise von 0,1 bis 20,0 Gew.-%, besonders bevorzugt von 0,2 bis 10,0 Gew.-% und insbesondere bevorzugt von 0,5 bis 5,0 Gew.-%.

An Hydroxysäuren können die kosmetischen, dermatologischen oder pharmazeutischen Formulierungen vorzugsweise Milchsäure, Glykolsäure, Salicylsäure, Zitronensäure oder Polyglykoldisäuren in freier oder teilweiser Neutralisation enthalten. Weiterhin können kosmetische, dermatologische oder pharmazeutische Formulierungen enthaltend Vitamin C oder Vitamin C-Derivate, Dihydroxyaceton oder Skin-whitening Actives wie Arbutin oder Glycyrrhetinsäure und deren Salze stabilisiert werden. Der Gehalt an einer oder mehreren dieser soeben genannten Substanzen, bezogen auf das Gesamtgewicht der Formulierungen, ist vorzugsweise von 0,1 bis 20,0 Gew.-%, besonders bevorzugt von 0,2 bis 10,0 Gew.-% und insbesondere bevorzugt von 0,5 bis 5,0 Gew.-%. Als Stabilisatoren können in den kosmetischen, dermatologischen oder pharmazeutischen Formulierungen Metallsalze von Fettsäuren, wie z. B. Magnesium-, Aluminium- und/oder Zinkstearat eingesetzt werden, vorzugsweise in Mengen von 0,1 bis 10,0 Gew.-%, besonders bevorzugt von 0,5 bis 8,0 Gew.-% und insbesondere bevorzugt von 1,0 bis 5,0 Gew.-%, bezogen auf das Gesamtgewicht der Formulierungen.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVPdimethylaminoethylmethacrylat-Copolymer, Guar-hydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxy-propyldiethylentriamin; Polyaminopolyamid und kationische Chitinderivate, wie beispielsweise Chitosan.

Die kosmetischen, dermatologischen oder pharmazeutischen Formulierungen können einen oder mehrere der oben genannten kationischen Polymere in Mengen von vorzugsweise 0,1 bis 5,0 Gew.-%, besonders bevorzugt von 0,2 bis 3,0 Gew.-% und insbesondere bevorzugt von 0,5 bis 2,0 Gew.-%, bezogen auf das Gesamtgewicht der Formulierungen, enthalten.

Des Weiteren können die kosmetischen, dermatologischen oder pharmazeutischen Formulierungen Filmbildner enthalten, die je nach Anwendungszweck ausgewählt sind aus Salzen der Phenylbenzimidazol-sulfonsäure, wasserlöslichen Polyurethanen, beispielsweise C₁₀-Polycarbamyl-polyglycerylester,Polyvinylalkohol, Polyvinylpyrrolidoncopolymeren, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich.

Die kosmetischen, dermatologischen oder pharmazeutischen Formulierungen können einen oder mehrere Filmbildner in Mengen von vorzugsweise 0,1 bis 10,0 Gew.-%, besonders bevorzugt von 0,2 bis 5,0 Gew.-% und insbesondere bevorzugt von 0,5 bis 3,0 Gew.-%, bezogen auf das Gesamtgewicht der Formulierungen, enthalten.

Die gewünschte Viskosität der Formulierungen kann durch Zugabe von Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

Die kosmetischen, dermatologischen oder pharmazeutischen Formulierungen können die Verdicker und Gelierungsmittel in Mengen von vorzugsweise 0,01 bis 20,0 Gew.%, besonders bevorzugt 0,1 bis 10,0 Gew.-%, insbesondere bevorzugt 0,2 bis 3,0 Gew.% und ganz besonders bevorzugt 0,4 bis 2,0 Gew.% enthalten, bezogen auf das Gesamtgewicht der Formulierungen.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden, bezogen auf das Gesamtgewicht der kosmetischen, dermatologischen oder pharmazeutischen Formulierungen.

An antimikrobiellen Wirkstoffen können Cetyltrimethylammoniumchlorid, Cetylpyridiniumchlorid, Benzethoniumchlorid, Diisobutylethoxyethyldimethyl-benzylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichloro-carbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox^{®}, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycin und Kombinationen dieser Wirksubstanzen zum Einsatz kommen.

Die kosmetischen, dermatologischen oder pharmazeutischen Formulierungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf das Gesamtgewicht der Formulierungen.

Die kosmetischen, dermatologischen oder pharmazeutischen Formulierungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol^{®}, Allantoin, Phytantriol^{®}, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die kosmetischen, dermatologischen oder pharmazeutischen Formulierungen können biogene Wirkstoffe bevorzugt in Mengen von 0,001 bis 5,0 Gew.-%, besonders bevorzugt von 0,01 bis 3,0 Gew.% und insbesondere bevorzugt von 0,1 bis 2,0 Gew.-%, bezogen auf das Gesamtgewicht der Formulierungen, enthalten.

Die kosmetischen, dermatologischen oder pharmazeutischen Formulierungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.-% und insbesondere bevorzugt in Mengen von 0,1 bis 10,0 Gew.-%, bezogen auf das Gesamtgewicht der Formulierungen, enthalten.

Als deodorierende Stoffe bevorzugt sind Allantoin und Bisabolol. Diese werden vorzugsweise in Mengen von 0,0001 bis 10,0 Gew.-% eingesetzt, bezogen auf das Gesamtgewicht der kosmetischen, dermatologischen oder pharmazeutischen Formulierungen.

Die kosmetischen, dermatologischen oder pharmazeutischen Formulierungen können als Pigmente/Mikropigmente sowie als anorganische Sonnenschutzfilter mikrofeines Titandioxid, Glimmer-Titanoxid, Eisenoxide, Glimmer-Eisenoxid, Zinkoxid, Siliciumoxide, Ultramarinblau, Chromoxide enthalten.

Die kosmetischen, dermatologischen oder pharmazeutischen Formulierungen können einen oder mehrere organische Sonnenschutzfilter enthalten, vorzugsweise ausgewählt aus 4-Aminobenzoesäure, 3-(4'-Trimethylammonium)-benzyliden-boran-2-on-methylsulfat, Camphor Benzalkonium Methosulfat, 3,3,5-Trimethyl-cyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäure-isoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-(('1,1-dimethylethyl)-amino-carbonyl)phenylamino]-1,3,5-triazin-2,4-y1)diimino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalzvon 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzirnidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2-Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Die Menge der vorgenannten Sonnenschutzfilter (eine oder mehrere Verbindungen) in den kosmetischen, dermatologischen oder pharmazeutischen Formulierungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der Formulierungen.

Die kosmetischen, dermatologischen oder pharmazeutischen Formulierungen können einen oder mehrere Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z. B. Selenmethionin), Stilbenen und deren Derivaten (z. B. Stilbenoxid, trans-Stilbenoxid), Superoxid-Dismutase und geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den kosmetischen, dermatologischen oder pharmazeutischen Formulierungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der Formulierungen.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenol und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.-% und besonders bevorzugt von 0,5 bis 5,0 Gew.-%, bezogen auf das Gesamtgewicht der Formulierungen.

Zusätzlich können die kosmetischen, dermatologischen oder pharmazeutischen Formulierungen organische Lösungsmittel enthalten. Prinzipiell kommen als organische Lösungsmittel alle ein- oder mehrwertigen Alkohole in Betracht.

Bevorzugt werden Alkohole mit 1 bis 4 Kohlenstoffatomen wie Ethanol, Propanol, Isopropanol, n-Butanol, i-Butanol, tert.-Butanol, Glycerin und Mischungen aus den genannten Alkoholen eingesetzt. Weitere bevorzugte Alkohole sind Polyethylenglykole mit einer relativen Molekülmasse unter 2000. Weitere geeignete Lösungsmittel sind beispielsweise Triacetin (Glycerintriacetat) und 1-Methoxy-2-propanol. Organische Lösungsmittel können in Mengen von vorzugsweise 0 bis 20,0 Gew.% und besonders bevorzugt 0,1 bis 20,0 Gew.-% eingesetzt werden, bezogen auf das Gesamtgewicht der Formulierungen.

Die kosmetischen, dermatologischen oder pharmazeutischen Formulierungen können ein oder mehrere Substanzen ausgewählt aus Farbmitteln, z. B. Farbstoffe und/oder Pigmente, enthalten. Die in den Formulierungen enthaltenen Farbstoffe und/oder Pigmente, sowohl organische als auch anorganische Farbstoffe und Pigmente, sind aus der entsprechenden Positivliste der Kosmetikverordnung bzw. der EG-Liste kosmetischer Färbemittel ausgewählt.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Suffo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1 -Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z. B. Paprikaextrakte, β-Carotin und Cochenille.

Vorteilhaft eingesetzt werden auch Perlglanzmittel wie z. B. Perlglanzpigmente, z. B. Fischsilber (Guanin/ Hypoxanthin-Mischkristalle aus Fischschuppen) und Perlmutt (vermahlene Muschelschalen), monokristalline Perlglanzpigmente wie z. B. Bismuthoxychlorid (BiOCl), Schicht-Substrat Pigmente, z. B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂O₃, TiO₂/Cr₂O₃, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCl), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2 - 50 µm, 5 - 25 µm, 5 - 40 µm, 5 - 60 µm, 5 - 95 µm, 5 -100 µm, 10 - 60 µm, 10 - 100 µm, 10 - 125 µm, 20 - 100 µm, 20 - 150 µm, sowie < 15 µm. Eine breitere Teilchengrößenverteilung, z. B. von 20 - 150 µm, ruft glitzernde Effekte hervor, während eine engere Teilchengrößenverteilung von < 15 µm für eine gleichmäßige seidige Erscheinung sorgt.

Die kosmetischen, dermatologischen oder pharmazeutischen Formulierungen enthalten Effektpigmente vorzugsweise in Mengen von 0,1 bis 20,0 Gew.-%, besonders bevorzugt von 0,5 bis 10,0 Gew.-% und insbesondere bevorzugt von 1,0 bis 5,0 Gew.-%, bezogen auf das Gesamtgewicht der Formulierungen.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Sofern die kosmetischen, dermatologischen oder pharmazeutischen Formulierungen perlglanzgebende Verbindungen enthalten, sind diese bevorzugt in einer Menge von 0,1 bis 15,0 Gew.-% und besonders bevorzugt in einer Menge von 1,0 bis 10,0 Gew.% in den Formulierungen enthalten, jeweils bezogen auf das Gesamtgewicht der Formulierungen.

Als Duftstoffe wie z. B. Duft- oder Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die Menge an Wasser in den kosmetischen, dermatologischen oder pharmazeutischen Formulierungen beträgt bevorzugt von 5 bis 95 Gew.-%, besonders bevorzugt von 40 bis 94 Gew.-% und insbesondere bevorzugt von 50 bis 90 Gew.-%, bezogen auf das Gesamtgewicht der Formulierungen.

Als Säuren oder Laugen zur pH-Wert Einstellung werden vorzugsweise Mineralsäuren, insbesondere HCl, anorganische Basen, insbesondere NaOH oder KOH, und organische Säuren, insbesondere Zitronensäure, verwendet.

Die kosmetischen, dermatologischen oder pharmazeutischen Formulierungen haben pH-Werte von vorzugsweise 2 bis 10, besonders bevorzugt von 3 bis 9, insbesondere bevorzugt von 4,5 bis 8 und außerordentlich bevorzugt von 5,5 bis 7,5.

In einer bevorzugten Ausführungsform der Erfindung sind die kosmetischen, dermatologischen oder pharmazeutischen Formulierungen Hautreinigunsmittel, Hautpflegeprodukte oder Haarbehandlungsmittel.

Bei den Hautreinigungsprodukten kann es sich um Cremeduschen, Gesichtsreinigungsmittel oder Badezusätze handeln.

Bei den Hautpflegeprodukten kann es sich um Cremes, Lotionen, Sprühemulsionen, Fluides oder Hydrodispersionen handeln.

Die kosmetischen, dermatologischen oder pharmazeutischen Formulierungen können in der Form von Sonnenschutzmitteln vorliegen und einen oder mehrere anorganische und/oder organische Sonnenschutzfilter enthalten.

Die Haarbehandlungsmittel können beispielsweise in Form von Haarkonditioniermitteln wie Cremespülungen und Haarkuren, die zur Verbesserung des Glanzes, der Kämmbarkeit und zum Hitzeschutz der Haare eingesetzt werden, vorliegen. Diese Haarbehandlungsmittel können ein oder mehrere kationische Tenside wie Cetrimoniumchlorid, Behentrimoniumchlorid, Stearamidopropyldimethylamin, Behenamidopropyldimethylamin oder quartäre Ammoniumverbindungen auf Esterbasis und/oder ein oder mehrere Silikone enthalten.

Die kosmetischen, dermatologischen oder pharmazeutischen Formulierungen können auch in Form von dekorativen Kosmetika wie z. B. Foundations oder Mascara, vorliegen.

Die kosmetischen, dermatologischen oder pharmazeutischen Formulierungen können in Form von Emulsionen vorliegen, beispielsweise als Emulsionen vom Typ Wasser-in-Öl oder Öl-in-Wasser. Unter den Emulsionen sind die Emulsionen vom Typ Öl-in-Wasser bevorzugt.

Der nichtwässrige Anteil der Emulsionen, der sich weitgehend aus dem Emulgator- und dem Ölkörper zusammensetzt, liegt vorzugsweise bei 5 bis 95 Gew.-% und besonders bevorzugt bei 15 bis 75 Gew.-%, bezogen auf das Gesamtgewicht der Emulsionen. Das bedeutet, dass die Emulsionen, bezogen auf ihr Gesamtgewicht, vorzugsweise 5 bis 95 Gew.-% und besonders bevorzugt 25 bis 85 Gew.-% wässrige Phase enthalten können, abhängig davon, ob Lotionen mit einer vergleichsweise niedrigen oder Cremes und Salben mit hoher Viskosität hergestellt werden sollen.

Die Herstellung der Emulsionen erfolgt bevorzugt in der Weise, dass man die Bestandteile der Ölphase zusammen mit der erfindungsgemäßen Zusammensetzung erwärmt und homogen vermischt. Die Bestandteile der Wasserphase werden ebenfalls vermischt und Öl- und Wasserphase werden dann unter intensivem Rühren miteinander gemischt. Anschließend wird der Ansatz kaltgerührt.

Die Herstellung der Emulsionen kann in an sich bekannter Weise, d. h. beispielsweise durch Heiß/Heiß-, Heiß/Kalt-, Kalt/Kalt- bzw. PIT-Emulgierung erfolgen.

Die erfindungsgemäßen Zusammensetzungen selbst sind in vorteilhafter Weise als Emulgator geeignet.

Ein weiterer Gegenstand der vorliegenden Erfindung betrifft daher die Verwendung der erfindungsgemäßen Zusammensetzungen als Emulgator.

Bevorzugt werden die erfindungsgemäßen Zusammensetzungen als Emulgatoren für kosmetische, dermatologische oder pharmazeutische Emulsionen verwendet, besonders bevorzugt für Emulsionen vom Typ Öl-in-Wasser. Hierbei werden die erfindungsgemäßen Zusammensetzungen vorzugsweise in Mengen von 0,1 bis 5 Gew.-% und besonders bevorzugt in Mengen von 0,3 bis 3 Gew.-%, bezogen auf das Gesamtgewicht der Emulsionen, verwendet.

Die nachfolgenden Beispiele und Anwendungen sollen die Erfindung näher erläutern, ohne sie jedoch darauf zu beschränken. Bei allen Prozentangaben handelt es sich um Gewichtsprozent (Gew.-%), sofern nicht explizit anders angegeben.

Vergleichsbeispiel A: Herstellung eines teilneutralisierten Phosphorsäureesters nach DE 197 07 800

95 g Mono Di Octyldodecylphosphat werden in einem 1-Liter-Kolben mit Rührer, Temperaturfühler, Rückflusskühler, Tropftrichter und Einleitungsmöglichkeit für N₂ vorgelegt. Bei einer Innentemperatur von 80 °C werden über den Tropftrichter 20,2 g einer 50 Gew.-%igen wässrigen Kaliumhydroxidlösung zugegeben. Bei Zugabe der Kaliumhydroxidlösung entsteht eine bei Raumtemperatur inhomogene, trübe hochviskose, schwer handhabbare Paste. Die Viskosität der Paste ließ sich nicht bestimmen.

### Beispiel A: Herstellung einer erfindungsgemäßen Zusammensetzung

95 g Mono Di Octyldodecylphosphat, mit einem Gewichtsverhältnis von Phosphorsäuremonoalkylester zu Phosphorsäuredialkylester von 1,15 zu 1,00 und einer Säurezahl von 177 mg KOH/g, und 95 g Sorbitanmonolaurat werden in einem 1-Liter-Kolben mit Rührer, Temperaturfühler, Rückflusskühler, Tropftrichter und Einleitungsmöglichkeit für N₂ vorgelegt. Bei einer Innentemperatur von 80 °C werden über den Tropftrichter 20,2 g einer 50 Gew.-%igen wässrigen Kaliumhydroxidlösung zugegeben. Die Mischung wird bei zunehmenden Verbrauch an KOH-Lösung klarer.

Anschließend wird das Gemisch 1 Stunde bei 70 °C nachgerührt und anschließend bei 60°C abgefüllt. Es wird eine klare Flüssigkeit mit einer Viskosität von 1700 mPa · s (Brookfield Viskosimeter, 20 °C, Spindel 5), einem pH-Wert von 7,2 (1 Gew.-% in Wasser bei 20°C) und einem Wassergehalt von 5,8 Gew.-% (Karl-Fischer) erhalten. Der Stockpunkt dieser Flüssigkeit beträgt 0 °C.

Der Wassergehalt des Produktes kann bis auf maximal 10 Gew.-% erhöht werden, wobei stehts eine klare Flüssigkeit erhalten wird.

### Beispiel B: Herstellung einer erfindungsgemäßen Zusammensetzung

95 g Mono Di Octyldodecylphosphat, mit einem Gewichtsverhältnis von Phosphorsäuremonoalkylester zu Phosphorsäuredialkylester von 1,15 zu 1,00 und einer Säurezahl von 177 mg KOH/g, und 95 g Sorbitanmonocaprylat werden in einem 1-Liter-Kolben mit Rührer, Temperaturfühler, Rückflusskühler, Tropftrichter und Einleitungsmöglichkeit für N₂ vorgelegt. Bei einer Innentemperatur von 80 °C werden über den Tropftrichter 20,2 g einer 50 Gew.-%igen wässrigen Kaliumhydroxidlösung zugegeben. Die Mischung wird bei zunehmenden Verbrauch an KOH-Lösung klarer.

Anschließend wird das Gemisch 1 Stunde bei 70 °C nachgerührt und anschließend bei 60°C abgefüllt. Es wird eine klare Flüssigkeit mit einer Viskosität von 2870 mPa · s (Brookfield Viskosimeter, 20 °C, Spindel 4), einem pH Wert von 6,2 (1 Gew.-% in Wasser bei 20°C) und einem Wassergehalt von 4,1 Gew.-% (Karl-Fischer) erhalten. Der Stockpunkt dieser Flüssigkeit beträgt -20 °C.

Der Wassergehalt des Produktes kann bis auf maximal 10 Gew.-% erhöht werden, wobei stehts eine klare Flüssigkeit erhalten wird.

### Analog Beispiel A werden folgende erfindungsgemäße flüssige

Zusammensetzungen hergestellt (Zusammensetzung Nr. 1 entspricht dem obigen Beispiel A, Zusammensetzung Nr. 3 entspricht dem obigen Beispiel B):

**Tabelle A Beispiele für erfindungsgemäße Zusammensetzungen**

| ZS Nr. | PSE | Gew.-Verhältnis von Phosporsäure Mono- zu Di-Alkylester | Säurezahl [mg KOH/g] | Menge PSE [g] | POE | Menge POE [g] | Menge 50 Gew.-%ige wässrige Kaliumhydroxidlösung [g] |
|---|---|---|---|---|---|---|---|
| 1 | Mono Di 2-Octyldodecylphosphat | 1,15 : 1,00 | 117 | 95,0 | Sorbitanmonolaurat | 95,0 | 95,0 |
| 2 | Mono Di 2-Octyldodecylphosphat | 1,00 : 1,02 | 174 | 36,0 | Sorbitanmonolaurat | 84,0 | 8,7 |
| 3 | Mono Di 2-Octyldodecylphosphat | 1,15 : 1,00 | 117 | 95,0 | Sorbitanmonocaprylat | 95,0 | 95,0 |
| 4 | Mono Di 2- Octyldodecylphosphat | 1,00 : 1,00 | 194 | 38,0 | Sorbitanmonocaprylat | 88,7 | 8,7 |
| 5 | Mono Di 2- Octyldodecylphosphat | 1,00 : 1,02 | 174 | 82,0 | Sorbitanmonocaprylat | 35,1 | 17,4 |
| 6 | Mono Di 2- Octyldodecylphosphat | 1,00 : 1,00 | 194 | 168,0 | Polyglyceryl- 3 Laurate | 72,0 | 36,8 |
| 7 | Mono Di 2- Octyldodecylphosphat | 1,07 : 1,00 | 183 | 55,0 | Polyglyceryl- 3 Laurate | 55,0 | 13,3 |
| 8 | Mono Di 2- Octyldodecylphosphat | 1,07 : 1,00 | 183 | 64,3 | Polyglyceryl- 3 Laurate | 150,3 | 14,9 |

| | | | | | | | |
|---|---|---|---|---|---|---|---|
| ZS = Zusammensetzung PSE = Phosphorsäureester POE = Polyolester Die Polyolester für die Zusammensetzungen Nr. 6, 7 und 8 wurden mit ihren INCI-Namen bezeichnet. Es handelt sich um die Monoester. | | | | | | | |

Folgende kosmetische Formulierungen wurden unter Verwendung von erfindungsgemäßen Zusammensetzungen hergestellt:

### Formulierungsbeispiel 1: Creme-Spülung

| | | |
|---|---|---|
| A | Genamin^{®} KDMP | 2,50 % |
| | *Behentrimonium Chloride* | |
| | Zusammensetzung Nr. 1 | 1,50 % |
| | SilCare^{®} Silicone 15M50 | 0,50 % |
| | *Phenyl Trimethicone* | |
| | Cetyl Alcohol | 3,00 % |
| | Jojoba Oil | 3,00 % |
| B | Wasser | ad 100 % |
| | Konservierungsmittel | q.s. |
| C | Duftstoff | 0,30 % |
| | Farbstoff | q.s. |
| D | NaOH (10 %ig)/ Zitronensäure (10 %ig) | q.s. |

### Herstellung:

I Schmelzen der Komponenten A bei 75 °C.
II Erwärmen von B auf 75 °C.
III II in I einrühren und kalt rühren.
IV C bei 30 °C zu III hinzugeben.
V pH-Wert auf 4 einstellen.

### Formulierungsbeispiel 2: O/W-Tagescreme mit UV-Filtern

| | | |
|---|---|---|
| A | Zusammensetzung Nr. 7 | 1,0 % |
| | Glyceryl Stearate | 0,5 % |
| | Cetearyl Alkohol | 0,5 % |
| | Mineralöl, niedrig viskos | 7,0 % |
| | Isopropyl Palmitate | 6,0 % |
| | SilCare^{®} Silicone 41M15 | 1,0% |
| | *Caprylyl Methicone* | |
| | Velsan^{®} CCT | 2.0 % |
| | *Caprylic*/*Capric Triglyceride* | |
| | Benzophenone - 3 | 1,0 % |
| B | Aristoflex^{®} AVC | 1,0 % |
| | *Ammonium Acryloyldimethyltaurate*/*VP Copolymer* | |
| C | Wasser | ad 100 % |
| | Glycerin | 5,0 % |
| | Tinosorb^{®} M | 3,0 % |
| | *Methylene Bis-Benzotriazolyl Tetramethylbutylphenol* | |
| | Allantoin | 0,3 % |
| | *Allantoin* | |
| D | Tocopheryl Acetate | 1,0 % |
| | Duftstoff | 0,3 % |
| | Nipaguard^{®} PDU | 1,0 % |
| | *Propylene Glycol (and) Diazolidinyl Urea (and)* | |
| | *Methylparaben (and) Propylparaben* | |

### Herstellung:

I Schmelzen der Komponenten A bei 70 °C.
II Erwärmen von C auf 70 °C.
III B in I einrühren und sofort anschließend II zugeben und kalt rühren.
IV D bei 30 °C zu III hinzugeben.

### Formulierungsbeispiel 3: Sprühbare Lotion

| | | |
|---|---|---|
| A | Zusammensetzung Nr. 1 bzw. 7 | 1,00 % |
| | Mineralöl, niedrig viskos | 8,00 % |
| | Isopropyl Palmitate | 3,00 % |
| | Cetearyl Alcohol | 0,50 % |
| | Velsan^{®} CCT | 2,00 % |
| | *Caprylic*/ *Capric Triglyceride* | |
| | Tegin^{®} M | 0,50 % |
| | *Glyceryl Stearate* | |
| | SilCare^{®} Silicone 41 M15 | 1,00 % |
| | *Caprylyl Methicone* | |
| B | Aristoflex^{®} AVC | 0,40 % |
| | *Ammonium Acryloyldimethyltaurate*/ *VP Copolymer* | |
| C | Wasser | ad 100 % |
| | Glycerin | 5,00 % |
| D | Duftstoff | 0,30 % |
| | Alcohol | 5,00 % |
| | Tocopheryl Acetate | 1,00 % |
| E | Nipaguard^{®} PDU | q.s. |
| | *Propylene Glycol (and) Diazolidinyl Urea (and)* | |
| | *Methylparaben (and) Propylparaben* | |

### Herstellung:

I Schmelzen der Komponenten A bei 70 °C, anschließend Zugabe von B.
II Erwärmen von C auf 70 °C.
III II in I einrühren und anschließend kalt rühren.
IV Die Komponenten D einzeln nacheinander bei 35 °C zu III zugeben.
V E zu IV zugeben und anschließend die Emulsion homogenisieren.

### Formulierungsbeispiel 4: O/W-Körperlotion

| | | |
|---|---|---|
| A | Zusammensetzung Nr. 1, 7 bzw. 8 | 0,50 % |
| | Glycine Soja Oil | 7,50 % |
| | Ethylhexyl Stearate | 7,50 % |
| B | Carbopol Ultrez 10 | 0,20 % |
| | *Carbomer* | |
| C | Wasser | ad 100 % |
| | Xanthan Gum | 0,10 % |
| | Glycerin | 2,80 % |
| | NaOH-Lösung, 10 %ig | 0,80 % |
| D | Tocopheryl Acetate | 0,50 % |
| | Phenonip^{®} | 0,80 % |
| | *Propylene Glycol (and) Diazolidinyl Urea (and) Methylparaben (and) Propylparaben* | |

### Herstellung:

I Gründliches Mischen der Bestandteile von A bei Raumtemperatur.
II Gründliches Mischen der Bestandteile von C bei Raumtemperatur.
III Einstreuen und Dispergieren von B in I.
IV II in III einrühren.
V Zugabe von D zu IV.

### Formulierungsbeispiel 5: Sprühbarer Sonnenschutz

| | | |
|---|---|---|
| A | Wasser | ad 100 % |
| | Allantoin | 0,20 % |
| | Glycerin | 0,50 % |
| B | Zusammensetzung Nr. 7 bzw. 8 | 1,80 % |
| | Velsan^{®} CCT | 1,00 % |
| | *Caprylic*/ *Capric Triglyceride* | |
| | SilCare^{®} Silicone | 5,00 % |
| | *Caprylyl Methicone* | |
| | Nipaguard^{®} PO5 | |
| | *Phenoxyethanol and Piroctone Olamine* | |
| C | Aristoflex^{®} AVC | 0,45 % |
| | *Ammonium Acryloyldimethyltaurate*/*VP Copolymer* | |
| D | Ethylhexyl Methoxycinnamate | 8,00 % |
| | Ethylhexyl Triazone | 4,50 % |
| | C12-15 Alkyl Benzoate | 2,00 % |
| E | Methylene Bis-Benzotriazolyl Tetramethylbutylphenol | 5,00 % |
| F | NaOH (10%ig)/ Zitronensäure (10%ig) | q.s. |

### Herstellung:

I Erhitzen von D bei 80-85 °C unter Rühren bis zur vollständigen Lösung.
II I bis auf 60 °C abkühlen lassen und die Komponenten B nacheinander zugeben und mischen.
III C in II unter Rühren einstreuen und dispergieren.
IV Herstellung einer Lösung aus den Komponenten A bei geringer Wärme (35-40 °C) und unter Rühren zu III geben.
V Anschließend E zu IV hinzugeben und gegebenenfalls den pH-Wert mit F einstellen.
VI 30 Minuten bei niedriger Umdrehungszahl (200 Umdrehungen pro Minute) rühren.

### Formulierungsbeispiel 6: Sonnenschutzcreme mit Titandioxide

| | | | |
|---|---|---|---|
| A | Zusammensetzung Nr. 2, 3 bzw. 6 | | 1,00 % |
| | Mineralöl, niedrigviskos | | 8,00 % |
| | Isopropyl Palmitat | | 3,00 % |
| | Velsan^{®}CCT | (Clariant) | 2,00 % |
| | *Caprylic*/*Capric Triglyceride* | | |
| | SilCare^{®} Silicone 41 M15 | (Clariant) | 1,00 % |
| | *Caprylyl Methicone* | | |
| | Glyceryl Stearate | | 0,50 % |
| | Cetearyl Alcohol | | 0,50 % |
| | Titan (IV) Oxide | | 8,00 % |
| | *Titanium Dioxide* | | |
| B | Aristoflex^{®} AVC | (Clariant) | 0,80 % |
| | *Ammonium Acryloyldimethyltaurate*/*VP Copolymer* | | |
| C | Glycerin | | 5,00 % |
| | Wasser | | ad 100 % |
| | Alcohol | | 1,00 % |
| D | Tocopheryl Acetate | | 1,00 % |
| | Nipaguard^{®} PDU | (Clariant) | q.s. |
| | *Propylene Glycol, Diazolidinyl Urea, Methylparaben,* | | |
| *Propylparaben* | | | |
| Fragrance | | | 0,30% |

### Herstellung:

I A bei 80 °C schmelzen, dann B zugeben.
II C auf 50 °C erwärmen.
III II in I einrühren und unter Rühren abkühlen.
IV D bei 35 °C zu III zugeben.

### Formulierungsbeispiel 7: Sonnenschutzcreme mit Zinkoxid

| | | | |
|---|---|---|---|
| A | Zusammensetzung Nr. 3 bzw. 7 | | 1,00 % |
| | Mineral Oil, low viscosity | | 8,00 % |
| | Isopropyl Palmitat | | 3,00 % |
| | Velsan^{®}CCT | (Clariant) | 2,00 % |
| | *Caprylic*/*Capric Triglyceride* | | |
| | Glycerylstearate | | 0,50 % |
| | Cetearylalkohol | | 0,50 % |
| B | Aristoflex^{®} AVC | (Clariant) | 0,80 % |
| | *Ammonium Acryloyldimethyltaurate*/*VP Copolymer* | | |
| C | Glycerin | | 5,00 % |
| | Alcohol | | 1,00 % |
| | Wasser | | ad 100 % |
| D | Tocopheryl Acetate | | 1,00 % |
| | Z-Cote HP1 | | 10,00 % |
| | *Zinc Oxide and Dimethicone* | | |
| | Preservative | | q. s. |
| E | Fragrance | | 0,30 % |

### Herstellung:

I A bei 70 °C schmelzen, dann B zugeben.
II C auf 40 °C erwärmen.
III II in I einrühren und unter Rühren abkühlen.
IV D bei 35 °C zu III zugeben.
V E zu IV zugeben.

### Formulierungsbeispiel 8: Feuchtigkeitsgebendes Styling-Gel

| | | | |
|---|---|---|---|
| A | Wasser | | ad 100 % |
| | Glycerin | | 3,00 % |
| | Aristoflex^{®} AVC | (Clariant) | 1,50 % |
| | *Ammonium Acryloyldimethyltaurate*/*VP Copolymer* | | |
| B | Mineral Oil | | 1,00 % |
| | Cetiol^{®} 868 | | 1,00 % |
| | *Octyl Stearate* | | |
| | SilCare Silicone^{®} Silicone 15M50 | (Clariant) | 1,50 % |
| | *Phenyl Trimethicone* | | |
| | Zusammensetzung Nr. 7 | | 1,00 % |
| C | Wasser | | 6,00 % |
| | Diaformer^{®} Z-712N | (Clariant) | 3,00 % |
| | *Acrylates*/*Lauryl Acrylate*/*Stearate Acrylate*/*Ethylamine* | | |
| | *Oxide Methacrylate Copolymer* | | |
| | Aloe Vera powder 200:1 | | 0,20 % |
| | Phenonip^{®} | (Clariant) | 0,40 % |
| | *Phenoxyethanol (and) Methylparaben (and) Ethylparaben (and)Butylparaben (and) Propylparaben (and) Isopropylparaben* | | |

### Herstellung:

I Komponenten A homogen vermischen.
II Mischung der Komponenten B zu I zugeben und homogenisieren.
III Mischung der Komponenten C zu II zugeben und homogenisieren.

### Formulierungsbeispiel 9: Leave-on conditioner lotion

| | | | |
|---|---|---|---|
| A | Wasser | | ad 100 % |
| | Glycerin | | 3,00 % |
| | Aristoflex^{®} AVC | (Clariant) | 1,00 % |
| | *Ammonium Acryloyldimethyltaurate*/*VP Copolymer* | | |
| B | Mineral Oil | | 1,00 % |
| | Cetiol^{®}868 | | 1,00 % |
| | *Octyl Stearate* | | |
| | SilCare^{®} Silicone 41 M15 | (Clariant) | 1,00 % |
| | *Caprylyl Methicone* | | |
| | Zusammensetzung Nr. 1 | | 1,00 % |
| | SilCare^{®} Silicone SEA | (Clariant) | 0,50 % |
| | *Trideceth-9 PG-Amodimethicone and Trideceth-12* | | |
| | Panthenol | | 0,50 % |
| C | Genamin^{®} KDMP | (Clariant) | 0,50 % |
| | *Behentrimonium Chloride* | | |
| D | NaOH (10 %ig)/ Zitronensäure (10 %ig) | | q.s. |

### Herstellung:

I Mischung der Komponenten A rühren bis ein homogenes Gel erhalten wird.
II Komponenten B mischen.
III C zu II zugeben und auf 70 °C bis zum Lösen erhitzen.
IV III zu I zugeben.
V pH-Wert auf 4 einstellen.

## Patentansprüche

1. Flüssige Zusammensetzung enthaltend
a) einen oder mehrere Phosphorsäurealkylester der Formel (I) 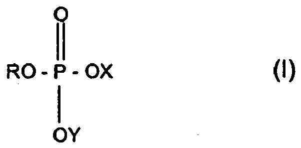 worin
R verzweigtes C₁₂-C₂₄-Alkyl bedeutet,
X H, Alkali oder Erdalkali bedeutet,
Y verzweigtes C₁₂-C₂₄-Alkyl, H, Alkali oder Erdalkali bedeutet,
und der Neutralisationsgrad der nicht veresterten Gruppen OX und OY gemeinsam (bezogen auf die Summe aller nicht veresterten Gruppen OX und OY) mindestens 25 mol-% ist, und
b) einen oder mehrere Polyolester, die erhältlich sind aus der Reaktion eines Polyols mit einer oder mehreren Säuren, wobei das Polyol ausgewählt ist aus gesättigten Polyolen bestehend aus Kohlenstoff-, Wasserstoff- und Sauerstoffatomen mit 3 bis 6 Kohlenstoffatomen und 2 bis 6 OH-Gruppen sowie Oligomeren davon mit im Mittel 1 bis 4 Wiederholungseinheiten und die eine oder die mehreren Säuren ausgewählt sind aus Carbonsäuren R¹-COOH, wobei R¹ ein linearer oder verzweigter gesättigter Alkylrest mit 7 bis 29 Kohlenstoffatomen oder ein linearer oder verzweigter ein- oder mehrfach ungesättigter Alkenylrest mit 7 bis 29 Kohlenstoffatomen ist und maximal 50 mol-% der OH-Gruppen des Polyols verestert sind, und
wobei die Komponenten a) und b) zusammen zu mindestens 80 Gew.-% in der Zusammensetzung enthalten sind, bezogen auf das Gesamtgewicht der Zusammensetzung, und das Gewichtsverhältnis von Verbindungen der Komponente a), bezogen auf die nicht neutralisierte Form, zu Verbindungen der Komponente b) von 12,5 : 87,5 bis 87,5 : 12,5 ist.

2. Zusammensetzung nach Anspruch 1, **dadurch gekennzeichnet, dass** in dem einen oder den mehreren Phosphorsäurealkylestern der Komponente a) R verzweigtes C₂₀-Alkyl bedeutet, X H oder Kalium bedeutet und Y verzweigtes C₂₀-Alkyl, H oder Kalium bedeutet.

3. Zusammensetzung nach Anspruch 1 oder 2, **dadurch gekennzeichnet, dass** in dem einen oder den mehreren Phosphorsäurealkylestern der Komponente a) R beta-verzweigtes Alkyl ist und Y, sofern es Alkyl bedeutet, ebenfalls beta-verzweigtes Alkyl ist.

4. Zusammensetzung nach einem oder mehreren der Ansprüche 1 bis 3, **dadurch gekennzeichnet, dass** das Gewichtsverhältnis von Phosphorsäuremonoalkylester zu Phosphorsäuredialkylester in der Komponente a) von 1 : 2 bis 2 : ist.

5. Zusammensetzung nach einem oder mehreren der Ansprüche 1 bis 4, **dadurch gekennzeichnet, dass** der eine oder die mehreren Polyolester der Komponente b) ausgewählt sind aus der Gruppe bestehend aus Sorbitanestern, Glycerinestern und Oligo- oder Polyglycerinestern.

6. Zusammensetzung nach einem oder mehreren der Ansprüche 1 bis 5, **dadurch gekennzeichnet, dass** der eine oder die mehreren Polyolester der Komponente b) ausgewählt sind aus der Gruppe bestehend aus Sorbitan Laurate, Sorbitan Isostearate, Sorbitan Diisostearate, Sorbitan Sesquiisostearate, Sorbitan Palmitate, Sorbitan Stearate, Sorbitan Distearate, Sorbitan Sesquistearate, Sorbitan Caprylate, Sorbitan Sesquicaprylate, Sorbitan Cocoate, Sorbitan Oleate, Sorbitan Dioleate, Sorbitan Sesquioleate, Sorbitan Palmate, Sorbitan Olivate, Glyceryl Laurate, Glyceryl Stearate, Polyglyceryl-2 Sesquiisostearate, Polyglyceryl-2 Caprate, Polyglyceryl-2 Caprylate, Polyglyceryl-2 Diisostearate, Polyglyceryl-2 Dioleate, Polyglyceryl-2 Distearate, Polyglyceryl-2 Isostearate, Polyglyceryl-2 Laurate, Polyglyceryl-2 Myristate, Polyglyceryl-2 Oleate, Polyglyceryl-2 Palmitate, Polyglyceryl-3 Caprate, Polyglyceryl-3 Caprylate, Polyglyceryl-3 Cocoate, Polyglyceryl-3 Dicocoate, Polyglyceryl-3 Diisostearate, Polyglyceryl-3 Dioleate, Polyglyceryl-3 Distearate, Polyglyceryl-3 Isostearate, Polyglyceryl-3 Laurate, Polyglyceryl-3 Myristate, Polyglyceryl-3 Oleate, Polyglyceryl-3 Palmitate, Polyglyceryl-4 Caprate, Polyglyceryl-4 Caprylate, Polyglyceryl-4 Dilaurate, Polyglyceryl-4 Laurate und Polyglyceryl-4 Oleate.

7. Zusammensetzung nach einem oder mehreren der Ansprüche 1 bis 6, **dadurch gekennzeichnet, dass** der Polyolester der Komponente b) Sorbitanmonolaurat oder Sorbitanmonocaprylat ist.

8. Zusammensetzung nach einem oder mehreren der Ansprüche 1 bis 7, **dadurch gekennzeichnet, dass** das Gewichtsverhältnis von Verbindungen der Komponente a), bezogen auf die nicht neutralisierte Form, zu Verbindungen der Komponente b) von 30 : 70 bis 70 : 30 ist.

9. Zusammensetzung nach einem oder mehreren der Ansprüche 1 bis 8, **dadurch gekennzeichnet, dass** sie, bezogen auf das Gesamtgewicht der Zusammensetzung, 0 bis 5 Gew.-% eines oder mehrerer Triester der Formel (II) 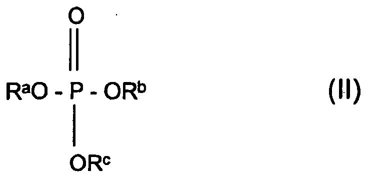 enthält, worin R^{a}, R^{b} und R^{c} jeweils unabhängig voneinander verzweigtes C₁₂-C₂₄-Alkyl bedeuten.

10. Zusammensetzung nach einem oder mehreren der Ansprüche 1 bis 9, **dadurch gekennzeichnet, dass** sie Wasser enthält.

11. Zusammensetzung nach Anspruch 10, **dadurch gekennzeichnet, dass** sie das Wasser in einer Menge von mindestens 2,0 Gew.-% enthält, bezogen auf das Gesamtgewicht der Zusammensetzung.

12. Zusammensetzung nach einem oder mehreren der Ansprüche 1 bis 11, **dadurch gekennzeichnet, dass** sie frei von ethoxylierten Verbindungen ist.

13. Verwendung einer Zusammensetzung nach einem oder mehreren der Ansprüche 1 bis 12 zur Herstellung einer kosmetischen, dermatologischen oder pharmazeutischen Formulierung.

14. Verwendung einer Zusammensetzung nach einem oder mehreren der Ansprüche 1 bis 12 als Emulgator.

## Claims

1. A liquid composition comprising
a) one or more phosphoric acid alkyl esters of the formula (I) 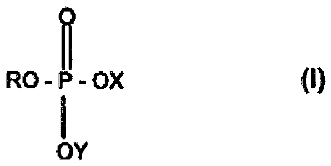 in which
R is branched C₁₂-C₂₄-alkyl,
X is H, alkali metal or alkaline earth metal,
Y is branched C₁₂-C₂₄-alkyl, H, alkali metal or alkaline earth metal,
and the degree of neutralization of the non-esterified groups OX and OY together (based on the sum of all non-esterified groups OX and OY) is at least 25 mol%, and
b) one or more polyol esters which are obtainable from the reaction of a polyol with one or more acids, where the polyol is selected from saturated polyols consisting of carbon, hydrogen and oxygen atoms having 3 to 6 carbon atoms and 2 to 6 OH groups, and oligomers thereof having on average 1 to 4 repeat units and the one or more acids are selected from carboxylic acids R¹-COOH, where R¹ is a linear or branched saturated alkyl radical having 7 to 29 carbon atoms or a linear or branched mono- or polyunsaturated alkenyl radical having 7 to 29 carbon atoms and at most 50 mol% of the OH groups of the polyol are esterified, and
where the components a) and b) together are present to at least 80% by weight in the composition, based on the total weight of the composition, and the weight ratio of compounds of component a), based on the non-neutralized form, to compounds of component b) is from 12.5 : 87.5 to 87.5 : 12.5.

2. The composition as claimed in claim 1, wherein, in the one or more phosphoric acid alkyl esters of component a), R is branched C₂₀-alkyl, X is H or potassium and Y is branched C₂₀-alkyl, H or potassium.

3. The composition as claimed in claim 1 or 2, wherein, in the one or more phosphoric acid alkyl esters of component a), R is beta-branched alkyl and Y, if it is alkyl, is likewise beta-branched alkyl.

4. The composition as claimed in one or more of claims 1 to 3, wherein the weight ratio of phosphoric acid monoalkyl ester to phosphoric acid dialkyl ester in component a) is from 1 : 2 to 2 : 1.

5. The composition as claimed in one or more of claims 1 to 4, wherein the one or more polyol esters of component b) are selected from the group consisting of sorbitan esters, glycerol esters and oligo- or polyglycerol esters.

6. The composition as claimed in one or more of claims 1 to 5, wherein the one or more polyol esters of component b) are selected from the group consisting of Sorbitan Laurate, Sorbitan Isostearate, Sorbitan Diisostearate, Sorbitan Sesquiisostearate, Sorbitan Palmitate, Sorbitan Stearate, Sorbitan Distearate, Sorbitan Sesquistearate, Sorbitan Caprylate, Sorbitan Sesquicaprylate, Sorbitan Cocoate, Sorbitan Oleate, Sorbitan Dioleate, Sorbitan Sesquioleate, Sorbitan Palmate, Sorbitan Olivate, Glyceryl Laurate, Glyceryl Stearate, Polyglyceryl-2 Sesquiisostearate, Polyglyceryl-2 Caprate, Polyglyceryl-2 Caprylate, Polyglyceryl-2 Diisostearate, Polyglyceryl-2 Dioleate, Polyglyceryl-2 Distearate, Polyglyceryl-2 Isostearate, Polyglyceryl-2 Laurate, Polyglyceryl-2 Myristate, Polyglyceryl-2 Oleate, Polyglyceryl-2 Palmitate, Polyglyceryl-3 Caprate, Polyglyceryl-3 Caprylate, Polyglyceryl-3 Cocoate, Polyglyceryl-3 Dicocoate, Polyglyceryl-3 Diisostearate, Polyglyceryl-3 Dioleate, Polyglyceryl-3 Distearate, Polyglyceryl-3 Isostearate, Polyglyceryl-3 Laurate, Polyglyceryl-3 Myristate, Polyglyceryl-3 Oleate, Polyglyceryl-3 Palmitate, Polyglyceryl-4 Caprate, Polyglyceryl-4 Caprylate, Polyglyceryl-4 Dilaurate, Polyglyceryl-4 Laurate and Polyglyceryl-4 Oleate.

7. The composition as claimed in one or more of claims 1 to 6, wherein the polyol ester of component b) is sorbitan monolaurate or sorbitan monocaprylate.

8. The composition as claimed in one or more of claims 1 to 7, wherein the weight ratio of compounds of component a), based on the non-neutralized form, to compounds of component b) is from 30 : 70 to 70 : 30.

9. The composition as claimed in one or more of claims 1 to 8, wherein it comprises, based on the total weight of the composition, 0 to 5% by weight of one or more triesters of the formula (II) 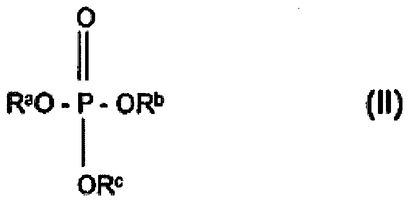 in which R^{a}, R^{b} and R^{c}, in each case independently of one another, are branched C₁₂-C₂₄-alkyl.

10. The composition as claimed in one or more of claims 1 to 9, wherein it comprises water.

11. The composition as claimed in claim 10, wherein it comprises the water in an amount of at least 2.0% by weight, based on the total weight of the composition.

12. The composition as claimed in one or more of claims 1 to 11, wherein it is free from ethoxylated compounds.

13. The use of a composition as claimed in one or more of claims 1 to 12 for producing a cosmetic, dermatological or pharmaceutical formulation.

14. The use of a composition as claimed in one or more of claims 1 to 12 as emulsifier.

## Revendications

1. Composition liquide contenant
a) un ou plusieurs phosphates d'alkyle de formule (I) 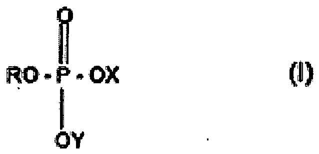 dans laquelle
R représente un groupe alkyle en C₁₂-C₂₄ ramifié,
X représente H, un atome de métal alcalin ou alcalino-terreux,
Y représente un groupe alkyle en C₁₂-C₂₄ ramifié, H, un atome de métal alcalin ou alcalino-terreux,
et le degré de neutralisation des groupes OX et OY non estérifiés ensemble (par rapport à la somme de tous les groupes OX et OY non estérifiés) est d'au moins 25 % en moles, et
b) un ou plusieurs esters de polyols, qui peuvent être obtenus à partir de la réaction d'un polyol avec un ou plusieurs acides, le polyol étant choisi parmi des polyols saturés constitués d'atomes de carbone, d'hydrogène et d'oxygène, comportant de 3 à 6 atomes de carbone et 2 à 6 groupes OH, ainsi que des oligomères de ceux-ci comportant en moyenne 1 à 4 motifs répétitifs et ledit un ou lesdits plusieurs acides étant choisis parmi des acides carboxyliques R¹-COOH, R¹ étant un radical alkyle saturé linéaire ou ramifié ayant de 7 à 29 atomes de carbone ou un radical alcényle linéaire ou ramifié, une ou plusieurs fois insaturé, ayant de 7 à 29 atomes de carbone et au maximum 50 % en moles des groupes OH du polyol étant estérifiés, et
les composants a) et b) étant ensemble contenus à raison d'au moins 80 % en poids dans la composition, par rapport au poids total de la composition, et le rapport pondéral des composés du composant a), sur la base de la forme non neutralisée, aux composés du composant b) valant de 12,5 : 87,5 à 87,5 : 12,5.

2. Composition selon la revendication 1, **caractérisée en ce que** dans ledit un ou lesdits plusieurs phosphates d'alkyle du composant a) R représente un groupe alkyle en C₂₀ ramifié, X représente H ou un atome de potassium et Y représente un groupe alkyle en C₂₀ ramifié, H ou un atome de potassium.

3. Composition selon la revendication 1 ou 2, **caractérisée en ce que** dans ledit un ou lesdits plusieurs phosphates d'alkyle du composant a) R représente un groupe alkyle bêta-ramifié et Y, s'il représente un groupe alkyle, est également un groupe alkyle bêta-ramifié.

4. Composition selon une ou plusieurs des revendications 1 à 3, **caractérisée en ce que** le rapport pondéral des phosphates de monoalkyle aux phosphates de dialkyle dans le composant a) vaut de 1 : 2 à 2 : 1.

5. Composition selon une ou plusieurs des revendications 1 à 4, **caractérisée en ce que** ledit un ou lesdits plusieurs esters de polyols du composant b) sont choisis dans le groupe constitué par des esters de sorbitanne, des esters de glycérol et des esters d'oligoglycérol ou de polyglycérol.

6. Composition selon une ou plusieurs des revendications 1 à 5, **caractérisée en ce que** ledit un ou lesdits plusieurs esters de polyols du composant b) sont choisis dans le groupe constitué par le laurate de sorbitanne, l'isostéarate de sorbitanne, le diisostéarate de sorbitanne, le sesquiisostéarate de sorbitanne, le palmitate de sorbitanne, le stéarate de sorbitanne, le distéarate de sorbitanne, le sesquistéarate de sorbitanne, le caprylate de sorbitanne, le sesquicaprylate de sorbitanne, le cocoate de sorbitanne, l'oléate de sorbitanne, le dioléate de sorbitanne, le sesquioléate de sorbitanne, le palmate de sorbitanne, l'olivate de sorbitanne, le laurate de glycéryle, le stéarate de glycéryle, le sesquiisostéarate de polyglycéryle-2, le caprate de polyglycéryle-2, le caprylate de polyglycéryle-2, le diisostéarate de polyglycéryle-2, le dioléate de polyglycéryle-2, le distéarate de polyglycéryle-2, l'isostéarate de polyglycéryle-2, le laurate de polyglycéryle-2, le myristate de polyglycéryle-2, l'oléate de polyglycéryle-2, le palmitate de polyglycéryle-2, le caprate de polyglycéryle-3, le caprylate de polyglycéryle-3, le cocoate de polyglycéryle-3, le dicocoate de polyglycéryle-3, le diisostéarate de polyglycéryle-3, le dioléate de polyglycéryle-3, le distéarate de polyglycéryle-3, l'isostéarate de polyglycéryle-3, le laurate de polyglycéryle-3, le myristate de polyglycéryle-3, l'oléate de polyglycéryle-3, le palmitate de polyglycéryle-3, le caprate de polyglycéryle-4, le caprylate de polyglycéryle-4, le dilaurate de polyglycéryle-4, le laurate de polyglycéryle-4 et l'oléate de polyglycéryle-4.

7. Composition selon une ou plusieurs des revendications 1 à 6, **caractérisée en ce que** l'ester de polyol du composant b) est le monolaurate de sorbitanne ou le monocaprylate de sorbitanne.

8. Composition selon une ou plusieurs des revendications 1 à 7, **caractérisée en ce que** le rapport pondéral des composés de composant a), sur la base de la forme non neutralisée, aux composés du composant b) vaut de 30 : 70 à 70 : 30.

9. Composition selon une ou plusieurs des revendications 1 à 8, **caractérisée en ce qu'**elle contient, par rapport au poids total de la composition, 0 à 5 % en poids d'un ou de plusieurs triesters de formule (II) 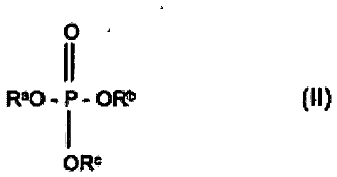 dans laquelle R^{a}, R^{b} et R^{c} représentent chacun indépendamment un groupe alkyle en C₁₂-C₂₄ ramifié.

10. Composition selon une ou plusieurs des revendications 1 à 9, **caractérisée en ce qu'**elle contient de l'eau.

11. Composition selon la revendication 10, **caractérisée en ce qu'**elle contient l'eau en une quantité d'au moins 2,0 % en poids, par rapport au poids total de la composition.

12. Composition selon une ou plusieurs des revendications 1 à 11, **caractérisée en ce qu'**elle est exempte de composés éthoxylés.

13. Utilisation d'une composition selon une ou plusieurs des revendications 1 à 12, pour la préparation d'une formulation cosmétique, dermatologique ou pharmaceutique.

14. Utilisation d'une composition selon une ou plusieurs des revendications 1 à 12, en tant qu'émulsifiant.
